**Europäisches Patentamt**

**European Patent Office**

**Office européen des brevets**

(19)

(11) Veröffentlichungsnummer: **0 205 629**
**A1**

(12) **EUROPÄISCHE PATENTANMELDUNG**

(21) Anmeldenummer: **85107490.6**

(22) Anmeldetag: **18.06.85**

(51) Int. Cl.⁴: **A 61 K 45/02, A 61 K 45/06**

(43) Veröffentlichungstag der Anmeldung: **30.12.86**
**Patentblatt 86/52**

(84) Benannte Vertragsstaaten: **AT BE CH DE FR GB IT LI LU NL SE**

(71) Anmelder: **BIOFERON Biochemische Substanzen GmbH & Co, Erwin-Rentschler-Strasse 21, D-7958 Laupheim 1 (DE)**

(72) Erfinder: **v. Eichborn, Johann-Friedrich, Dr., Humlangen 6, D-7901 Hüttisheim (DE)**
Erfinder: **Link, Franz, Dr., Am Kohlenschacht 5, D-8403 Bad Abbach (DE)**
Erfinder: **Obert, Hans-Joachim, Dr., Adolf-Gröber-Strasse 12, D-7958 Laupheim (DE)**

(74) Vertreter: **Dipl.-Ing. Schwabe, Dr. Dr. Sandmair, Dr. Marx, Stuntzstrasse 16 Postfach 86 02 45, D-8000 München 86 (DE)**

(54) Verwendung von Interferon-gamma (IFN-gamma) enthaltenden Präparationen zur Behandlung rheumatischer Erkrankungen.

㋟ Die Erfindung betrifft die Verwendung von Interferon-gamma enthaltenden Präparationen zur Behandlung rheumatischer Erkrankungen.

EP 0 205 629 A1

0205629

Verwendung von Interferon-gamma (IFN-gamma) enthaltenden
Präparationen zur Behandlung rheumatischer Erkrankungen

Alle bekannten Interferone werden auf Grund von Homologien
sowohl zwischen den Nukleotidsequenzen ihrer Strukturgene als
auch zwischen ihren Aminosäuresequenzen eindeutig in drei Gruppen
eingeteilt, die als IFN-alpha, IFN-beta und IFN-gamma bezeichnet
werden.

Die verwendeten Bezeichnungen folgen der neuesten Empfehlung
des "Interferon Nomenclature Commitee", die von J. Vilcek 1983
in Archives of Virology Vol. 77, S. 283-285, veröffentlicht
wurden. Zusätzlich zu den genannten Hauptkriterien kann zur
Charakterisierung der IFN-gamma-Gruppe herangezogen werden,
daß keine immunologische Kreuzreaktion mit den beiden anderen
Gruppen erfolgt sowie ihre biologische Instabilität bei pH 2
im Gegensatz zu IFN-alpha und IFN-beta.

IFN-gamma wird nach Stimulation der Gesamtpopulation der Leukozyten, nach der Art der Präparation üblicherweise als buffy
coats bezeichnet, durch Mitogene, Antigene oder spezifische
Antikörper in einem komplexen Prozeß zusammen mit einer großen
Anzahl weiterer Faktoren (Mediatoren) ausgeschüttet wie Interleukin 1 (IL 1), Interleukin 2 (IL 2), koloniestimulierender
Faktor (CSF), migrationsinhibierender Faktor (MIF), Makrophagen
aktivierender Faktor (MAF), Tumor nekrotisierender Faktor (TNF)
Lymphotoxin (LT) und Fibroblastenwachstums-Faktor (FGF). Weitere
Faktoren sind beschrieben von J. A. Georgiades et al. in Came,
P. E. und Carter, W.A. (eds.), Interferons and Their Applications,
Springer Verlag, 1984, und von Waksman, B. H. in Cohen, S.
et al. (eds.), Biology of the Lymphokines, Academic Press Inc.,
1979.

Diejenigen Faktoren, die von der Untergruppe der Lymphozyten
gebildet werden, bezeichnet man allgemein als Lymphokine. Zu
dieser Gruppe wird auch IFN-gamma gerechnet, das hauptsächlich
von T-Lymphozyten produziert wird. Die Syntheseleistung der
IFN-gamma produzierenden T-Lymphozyten wird ihrerseits wieder
von dem Vorhandensein der oben erwähnten Faktoren beeinflußt.

Weiterhin existieren etablierte bzw. transformierte Zellinien, die IFN-gamma konstitutiv produzieren, wie von N. Fujii et al. in J. Immunology 130, S. 1683-1686, und A. Zlotnik et al. in J. Immunology 131, S. 794-800, beschrieben.

Ein bevorzugtes Verfahren zur Gewinnung von humanem Roh-IFN-gamma wird nachfolgend beschrieben. Als Ausgangsmaterial dienen lymphozytenreiche Plasmafraktionen von Blutkonserven, sogenannte "buffy coats", die gepoolt und durch schonende Zentrifugation bei 600 - 800 g von Plasmaresten befreit werden. Die pelletierten Gesamtleukozyten werden in vorgewärmtem Medium mit einer Dichte von 5 Millionen Zellen/ml suspendiert. Die Zellsuspension wird in aliquots in geeigneten Kultivierungsgefäßen nach Zugabe eines Mitogens (z.B. Phythämagglutinin) und Phorbolester (z.B. Phorbolmyristylacetat (PMA) bis zu 70 Stunden bei 37 Grad C auf einem Schüttler inkubiert. Die IFN-gamma-haltigen Kulturüberstände werden durch Zentrifugation gewonnen und können bis zur Weiterverwendung bei 4 Grad C gelagert werden. Üblicherweise enthalten die so gewonnenen Präparationen etwa 10.000 Internationale Referenzeinheiten (I.E.) IFN-gamma pro ml.

Der IFN-gamma-Gehalt wird durch einen CPE-Reduktionstest in geeigneten Indikatorzellen (z.B. WISH) mit einem Testvirus (z.B. EMC der Maus) gegen den Referenzstandard Gg 23-901-530 des National Institute of Health (USA) als antivirale Aktivität des Präparates bestimmt.

Weitere Methoden zur Gewinnung von IFN-gamma-Präparationen werden von Y.K. Yip et al. in "Infection and Immunity", Okt. 1981, S. 131- 133, Vol. 34 und in den US-PS 4,376,821 und 4,376,822 sowie 4,460,685 beschrieben.

Ein grundsätzlich anderer Weg zur Gewinnung von humanen IFN-gamma-Präparaten wird mit der Einschleusung des humanen Strukturgens mit Hilfe geeigneter Vektoren in heterologe Wirtszellen beschritten. Diese rekombinanten Gene werden konstitutiv oder nach Zugabe spezifischer Induktoren von der Wirtszelle exprimiert.

Auch wenn es sich bei dem Strukturgen um eine eindeutig definierte Sequenz aus Nukleotiden handelt, die zwingend zur Synthese einer daraus ableitbaren Aminosäuresequenz führt, ist das IFN-gamma-Molekül des mit Hilfe der Wirtszelle hergestellten Präparates nicht notwendigerweise mit einem natürlich vorkommenden IFN-gamma identisch. Diese Möglichkeit der Variation unter Erhaltung der spezifischen Wirkung durch "post processing" wird durch das sogenannte Proteindesign erweitert, durch das das Strukturgen durch chemische bzw. biochemische Vollsynthese oder Modifikation verändert werden kann.

Als Wirtszellen zur Aufnahme des Humangens können außer Bakterien (z.B. E.coli wie von G. Simons et al. in Gene 28, S. 55-64, 1984, beschrieben) auch Hefen (z.B. Saccharomyces cerevisiae, wie von R. Derynck et al. in Nucleic Acids Research 11, S. 1819-1837, 1983, beschrieben) oder eukaryotische Zellen (wie z.B. Chinese Hamster Ovary Cells, wie von S. J. Scahill et al. in Proc. Nat. Acad. Sci. USA 80, S. 4654-4658, 1983, Affenzellen, wie von P. W. Gray et al. in Nature 295, S. 503-508, 1982, beschrieben) dienen.

In einigen dieser Systeme reichert sich entweder IFN-gamma bis zu einer Konzentration von mehr als 100.000 I.E. pro ml in der Kulturflüssigkeit an oder es wird im Wirt selbst angereichert und stellt dort bis zu 25 Prozent des Proteingehalts der Zelle dar.

Die Anreicherung bzw. Reinigung der nach den vorstehend beschriebenen Verfahren erhaltenen IFN-gamma-Präparationen kann nach den folgenden Methoden einzeln oder in Kombination erfolgen:

1. Controlled Pore Glass (CPG) oder Silica-Gel;

2. Gelfiltration (z.B. AcA 54 oder Sephacel S 200);

3. Ionenaustauschchromatographie (CM-Sepharose oder Phosphocellulose oder DEAE-cellulose);

4. Affinitätschromatographie (Con A-Sepharose oder Poly-U-Sepharose oder Cu-Chelat-Sepharose);

5. Immunaffinitätschromatographie (Anti-IFN-gamma-Sepharose);

6. HPLC (z.B. mit Reversed Phase Materialien).

Entsprechende Verfahren wurden von Y.K. Yip et al. "Partial Purification and Characterization of Human Gamma (Immune) Interferon"
in Proc. Nat. Acad. Sci. USA, 78, S. 1601-1605, 1981, oder von
D. Novick et al., EMBO Journal 2, S. 1527-1530, 1983, oder in
der DE-PS 3136166 A 1 beschrieben.

Durch geeignete Kombinationen ist eine Reinigung bis zur elektrophoretischen Homogenität möglich, z. Zt. liegt die dabei erreichte
maximale spezifische Aktivität bei 100 - 200 Millionen I.E., während
die beschriebenen Durchschnittswerte bei 10 und 50 Millionen I.E.
liegen.

Wie die anderen Interferone ist auch IFN-gamma eine Substanz mit
antiviraler, antiproliferativer und immunmodulierender Wirkung.
Aufgrund seiner in vitro stärkeren antiproliferativen Wirkung
als IFN-alpha und IFN-beta wurde es bisher nahezu ausschließlich bei Patienten mit malignen Erkrankungen eingesetzt (Came, P.E.
und Carter, W. A.: Interferons und their applications. Springer
Verlag: Berlin, Heidelberg, New York, Tokyo, 1984; De Maeyer, E. D.
und Schellekens, H. (Hrsg.): The biology of the interferon system
1983. Elsevier Science Publishers: Amsterdam, New York, Oxford,
1983).

Bei Rheuma handelt es sich um Schmerzen und Funktionseinschränkungen
in den Bewegungsorganen (Mathies, H: Rheuma. Gustav Fischer Verlag:
Stuttgart, New York 1983). Nach diagnostisch-therapeutisch-praktischen Gesichtspunkten werden unterschieden: entzündlicher Rheumatismus, degenerativer Rheumatismus und extraartikulärer Rheumatismus.
Zum rheumatischen Formenkreis werden weiterhin gezählt: systemischer
Lupus erythematodes, progressive systemische Sklerodermie, Dermatomyositis, Panarteriitis nodosa, Arthritis urica und Chondrokalzinose
(Pschyrembel, W.: Klinisches Wörterbuch. Walter de Gruyter: Berlin,
New York 1982). Bei der zum entzündlichen Rheumatismus zählenden
chronischen Arthritis wird die bei Kindern und Jugendlichen auftretende
Form häufig von der der Erwachsenen unterschieden (Anzell, B.M.:
Juvenile chronic polyarthritis. Arthr. Rheum. Suppl. 20, 176-180,
1977; Truckenbrodt, H.: Die juvenile chronische Arthritis und ihre
Subgruppen. Münch. med. Wschr. 126, 1076-1078, 1984).

Da die Ätiologie der weitaus meisten rheumatischen Erkrankungen
nicht geklärt ist, steht eine kausale Therapie in der Regel nicht
zur Verfügung (Krüger, K.: Neue Therapieprinzipien in der Rheumatologie. Münch. med. Wschr. 126, 1084-1086, 1984). In der medikamentösen Behandlung werden verschiedenartige Arzneimittel eingesetzt,
die zwei Gruppen zugeordnet werden können: die symptomatischen
Antirheumatika wie Salizylsäure, Indometacin und Glukokortikoide,
die rasch, aber nur so lange wirken, wie die Mittel genommen werden,
und die Basis-Antirheumatika wie Goldsalze, D-Penicillamin, Chloroquin und Immunsuppressiva, die in die Pathogenese eingreifen und
erst einige Wochen nach Beginn der Therapie wirksam sind, deren
Effekt aber auch noch nach Absetzen für einige Zeit anhält (Mathies, H. s.o.; Butler, R.C. und Goddard, D.H.: Controversy in the
treatment of rheumatoid arthritis. Lancet ii, 278-279, 1984).
Außerdem werden noch eine Reihe weiterer Substanzen in der Rheumatherapie eingesetzt wie z. B. die Thymushormone (Krüger, K.: s.o.).

Eine mit IFN-alpha durchgeführte Therapie stellte sich als
unwirksam gegen rheumatoide Arthritis heraus (Kajandar, A.
et al.: Interferon treatment of rheumatoid arthritis. Lancet
i, 984, 1979). Dieses Ergebnis ist nicht überraschend, da
bei bestimmten rheumatischen Erkrankungen einschließlich
der rheumatoiden Arthritis endogen Interferone gebildet werden,
denen keine therapeutische Wirksamkeit zugesprochen wird
oder die sogar für die Pathogenese dieser Erkrankungen mitverantwortlich gemacht werden. (Hooks, J.J. et al.: Immune interferon
in the circulation of patients with autoimmune disease. N.
Engl. J. Med. 301, 5-8, 1979; Preble, O.T. et al.: Systemic
lupus erythematosus: presence in human serum of an unusual
acid-labile leucocyte interferon. Science 216, 429-431, 1982;
Degré, M. et al.: Immune interferon in serum and synovial
fluid in rheumatoid arthritis and releated disorders. Ann.
Rheumatic Dis. 42, 672-676, 1983; Arvin, A.M. und Miller,
J.J.: Acid labile alpha-interferon in sera and synovial fluids
from patients with juvenile arthritis. Arthritis and Rheumatism
27, 582-585, 1984; Rosenbach T.O. et al.: Interferon triggers
experimental synovitis and may potentiate auto-immune disease
in humans. Clin. Rheumatol. 3, 361-364, 1984).

Im Rahmen eigener klinischer Prüfungen konnte bei der Behandlung verschiedener rheumatischer Erkrankungen jedoch unerwartet gefunden werden, daß sowohl eine natürliche als auch
eine rekombinante Interferon-gamma enthaltende Präparation
nach wenigen Injektionen die Schmerzen anhaltend beseitigte
und die Motorik der Patienten erheblich verbesserte. Interferon-gamma war therapeutisch wirksam bei entzündlichem Rheumatismus (Beispiel 1-3), extraartikulärem Rheumatismus (Beispiel 4 u. 5) und degenerativem Rheumatismus (Beispiel 6).
Diese Ergebnisse überraschen, da die bisher klinisch gesicherten Wirkungen der Interferone dies nicht vermuten ließen und
Interferon bei diesen Erkrankungen sogar als kontraindiziert
angesehen wurde.

Natürliches IFN-gamma und rekombinantes IFN-gamma können wie folgt dosiert werden: intravenös als Bolus oder bis zu einer 24-stündigen Dauerinfusion, intramuskulär (einschließlich paravertebral bzw. periartikulär), intrasynovial, intraartikulär, periostal, subkutan, intrakutan, intrathekal, oral oder topisch, z.B. in einer Salben- oder Gelgrundlage durch Auftragen auf oder Einmassieren in die Haut. Bei diesen Applikationsformen können auch die dem Fachmann bekannten Depotformen verwendet werden.

Bei allen Applikationen werden je nach Schwere der Erkrankung gegeben: 200 Internationale Einheiten (I.E.) bis 2.000 Millionen I.E. ein- oder mehrmalig an einem Tag oder an mehreren Tagen. Bei mehrtägiger Gabe können ein oder mehrere Dosen

a) an aufeinanderfolgenden Tagen
b) alle 2 bis 6 Tage
c) einmal pro Woche
d) alle zwei bis vier Wochen
e) einmal pro Monat oder
f) jedesmal bei Auftreten von Schmerzen

verabreicht werden.

Für die praktische Anwendung empfiehlt es sich, die Behandlung mit niedrigen Dosen zu beginnen, beispielsweise mit Dosen von 20.000 bis 2.000.000 I.E. bzw. ca. 2 µg bis 200 µg IFN-gamma.

Zur Steigerung der Effektivität der Interferon-gamma-Präparation können folgende Substanzen zusätzlich gegeben werden:

a) andere Interferone und/oder andere von Leukozyten gebildete bzw. durch gentechnologische Verfahren hergestellte Zell-Mediatoren wie IL 1, IL 2, CSF, MIF, MAF, TNF, LT und FGF.

b) in der Therapie bisher verwendete Antirheumatika einschließlich der Thymushormone.

Zur Herstellung der jeweiligen Darreichungsform werden die dem Fachmann bekannten Hilfsstoffe verwendet.

- 8 -

B e i s p i e l   1

| Patient: | Lu. männl. |
|---|---|
| Diagnose: | schwere chronische Polyarthritis seit über 10 Jahren, Deformation an Händen und Füßen, bei Einlieferung in die Klinik nicht bewegungsfähig, bisher keine Besserung des Leidens möglich. |
| Substanz: | humane IFN-gamma-Präparation aus menschlichen Leukozyten |
| Applikationsweise: | subkutan |

Therapieschema:

| Behandlungstag | Dosierung |
|---|---|
| 1. | $0,1 \times 10^6$ I.E. |
| 2. | $0,5 \times 10^6$ I.E. |
| 3. | $0,5 \times 10^6$ I.E. |
| 4. | $1,0 \times 10^6$ I.E. |
| 5. | $1,0 \times 10^6$ I.E. |
| 8. | $1,5 \times 10^6$ I.E. |
| 9. | $1,5 \times 10^6$ I.E. |
| 10. | $1,5 \times 10^6$ I.E. |
| 11. | $1,5 \times 10^6$ I.E. |

Ergebnis: Am 6. Behandlungstag zeigte sich eine wesentliche Besserung der Schmerzen in den unteren Extremitäten; ab dem 11. Behandlungstag war der Patient völlig schmerzfrei. Er war gehfähig und konnte sogar Treppen steigen. Die Klopfempfindlichkeit war verschwunden.

B e i s p i e l   2

| Patient: | Zi. männl. |
|---|---|
| Diagnose: | schwere chronische Polyarthritis mit Deformation an beiden Händen |

0205629

Substanz: humane IFN-gamma-Präparation aus menschlichen Leukozyten

Applikationsweise: subkutan

Therapieschema:

| Behandlungstag | Dosierung |
|---|---|
| 1. | $0,5 \times 10^6$ I.E. |
| 2. | $0,5 \times 10^6$ I.E. |
| 3. | $0,5 \times 10^6$ I.E. |
| 4. | $0,5 \times 10^6$ I.E. |
| 5. | $0,5 \times 10^6$ I.E. |
| 8. | $1,0 \times 10^6$ I.E. |
| 9. | $1,0 \times 10^6$ I.E. |
| 10. | $1,0 \times 10^6$ I.E. |

Ergebnis: Unter der Therapie mit $0,5 \times 10^6$ I.E. trat
eine zunehmende Besserung der Schmerzen
ein, unter der Therapie mit $1,0 \times 10^6$ I.E.
wurde der Patient dann völlig schmerzfrei.
Der Patient konnte zu Beginn der Therapie
maximal 4 Minuten auf einem Heimtrainer
radfahren, ab dem 9. Behandlungstag
2 x 20 Minuten. Der Patient fühlt sich
subjektiv wohl und benötigt kein Cortison
mehr. Er wurde am 17. Tag nach Behandlungsbeginn aus der Klinik entlassen. Der Patient
war am Entlassungstag schmerzfrei, seine
Beweglichkeit einwandfrei, über den
Gelenken existierte keine Klopfempfindlichkeit mehr.

B e i s p i e l   3

Patient: I. Jä. weibl.

Diagnose: seronegative rheumatische Polyarthritis,
Anämie, lokal nodale Hyperplasie der Leber

Substanz: humane IFN-gamma-Präparation aus E. coli

Applikationsweise: intramuskulär

| Therapieschema: | Behandlungstag | Dosierung |
|---|---|---|
| | 1. | 100 µg Wirkstoff |
| | 2. | 100 µg Wirkstoff |
| | 3. | 100 µg Wirkstoff |
| | 4. | 100 µg Wirkstoff |
| | 5. | 100 µg Wirkstoff |
| | 6. | 100 µg Wirkstoff |
| | 7. | 100 µg Wirkstoff |
| | 8. | 100 µg Wirkstoff |
| | 11. | 175 µg Wirkstoff |
| | 12. | 175 µg Wirkstoff |
| | 13. | 50 µg Wirkstoff |
| | 14. | 50 µg Wirkstoff |

Ergebnis:       Nachdem sich in den ersten Therapietagen die Schmerzen verstärkten, war die Patientin am 4. Behandlungstag erstmals schmerzfrei. Im weiteren Verlauf der Therapie traten die Schmerzen nur noch kurzfristig auf. Seit kurzem erhält die Patientin einmal pro Woche 10 µg Wirkstoff i.m. Die Patientin verträgt das Medikament gut und ist seither wieder schmerzfrei.

### B e i s p i e l   4

Patient:       H. L. weibl.

Diagnose:       gesicherte rheumatoide Arthritis seit 4 Jahren. Betroffen sind Hände, Hüften, Knie, Sprunggelenke und Zehengrundgelenke. Patientin refraktär gegen Gold und Prednisolon.

Substanz:       humane IFN-gamma-Präparation aus E.coli

Applikation:       subcutan

Dosis:       2 mal 250 µg täglich als Initialtherapie; 2 mal 50 µg täglich als Erhaltungstherapie.

Ergebnis:          Nach 5 Tagen Behandlung war die Patientin
                   schmerzfrei und frei beweglich. Unter der
                   Erhaltungstherapie besserte sich das
                   klinische Bild der rheumatoiden Arthritis
                   weitgehend und anhaltend. Die Patientin
                   bleibt schmerzfrei.


                   B e i s p i e l     5


Patient:           W.D. weibl.

Diagnose:          Periarthritis humeroscapularis seit
                   1 Woche

Substanz:          humane IFN-gamma-Präparation aus menschlichen Leukozyten

Applikationsweise und
Therapieschema:    einmalig subkutan $(0,5 \times 10^6$ I.E.)

Ergebnis:          Die Patientin war innerhalb weniger
                   Minuten schmerzfrei. Seither sind keine
                   Schmerzen mehr aufgetreten.


                   B e i s p i e l     6


Patient:           L. L.  männl.

Diagnose:          Ischialgie $L_5/S_1$ rechts, Osteoporose
                   seit 3 Wochen

Substanz:          humane IFN-gamma-Präparation aus menschlichen Leukozyten

Applikationsweise: paravertebral

Therapieschema:    Behandlungstag       Dosierung
                        1.               $0,5 \times 10^6$ I.E.
                        21.              $0,5 \times 10^6$ I.E.

Ergebnis:          Nach der ersten Injektion war der
                   Patient 5 Tage lang, nach der zweiten
                   Injektion bis heute (9 Monate nach
                   erstem Behandlungstag) schmerzfrei.

Patentansprüche

1. Verwendung von Interferon-gamma enthaltenden Präparationen zur Behandlung rheumatischer Erkrankungen.

2. Verwendung nach Anspruch 1 zur Behandlung von entzündlichem Rheumatismus.

3. Verwendung nach Anspruch 2 zur Behandlung von chronischer Polyarthritis (rheumatoider Arthritis).

4. Verwendung nach Anspruch 2 zur Behandlung von juveniler chronischer Arthritis.

5. Verwendung nach Anspruch 2 zur Behandlung der Psoriasis anthropathica.

6. Verwendung nach Anspruch 1 zur Behandlung von extraartikulärem Rheumatismus (Weichteilrheumatismus).

7. Verwendung nach Anspruch 6 zur Behandlung von Muskelrheumatismus und Periarthritis humeroscapularis.

8. Verwendung nach Anspruch 1 zur Behandlung von degenerativem Rheumatismus.

9. Verwendung nach Anspruch 1 zur Behandlung von Lupus erythematodes.

10. Verwendung nach Anspruch 1 zur Behandlung von Dermatomyositis.

11. Verwendung nach Anspruch 1 zur Behandlung von Sklerodermie.

12. Verwendung nach Anspruch 1, dadurch gekennzeichnet, daß das Interferon-gamma von menschlichen Zellen oder durch gentechnologische Verfahren erhalten wurde.

13. Verwendung nach Anspruch 1, dadurch gekennzeichnet, daß die Interferon-gamma enthaltende Präparation zusätzlich andere Interferone und/oder andere durch Leukozyten gebildete bzw. durch gentechnologische Verfahren hergestellte Zell-Mediatoren enthält.

14. Verwendung von Interferon-gamma enthaltenden Präparationen nach einem der Ansprüche 1 bis 11, dadurch gekennzeichnet, daß die Präparation in einer intravenös, intramuskulär, intraartikulär, intrasynovial, periostal, subkutan, intrakutan, intrathekal, oral oder topisch zu verabreichenden Applikationsform vorliegt.

15. Verwendung nach Anspruch 14, dadurch gekennzeichnet, daß die dem Fachmann für Polypeptidtherapeutika bekannten Depotformen verabreicht werden.

16. Verwendung von Interferon-gamma enthaltenden Präparationen nach einem der Ansprüche 1 bis 15, dadurch gekennzeichnet, daß die verabreichte Einzeldosis 200 Internationale Referenzeinheiten (I.E.) bis 2.000 Millionen I.E. (entspr. ca. 20 ng bis 20 mg) Interferon-gamma enthält.

17. Verwendung nach einem der Ansprüche 1 - 16, dadurch gekennzeichnet, daß die Präparationen zusätzlich symptomatisch wirkende Antirheumatika, Basisantirheumatika und/oder Thymus-Hormone enthalten.

Europäisches
Patentamt

**EUROPÄISCHER RECHERCHENBERICHT**

Nummer der Anmeldung

EP  85 10 7490

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | US-A-4 483 849  (W.A. CARTER et al.) <br> *  Titelblatt,  Zusammenfassung, letzter  Satz;  Spalte  2,  Zeilen 54-68 * | 1,11-17 | A 61 K  45/02 <br> A 61 K  45/06 |
| X | EP-A-0 077 063  (VIRAL GENETICS INC.) <br> *  Ansprüche  1,10,11;  Seite  6, Zeilen 20-25; Seite 8, Zeile 27 - Seite  9,  Zeile  12;  Seite  13, Zeilen  25-31;  Seite  14, Zeilen 11-20; Seite 25, Zeile 25 - Seite 26, Zeile 7 * | 1,2,5, 12-17 | |
| X | CHEMICAL ABSTRACTS, Band 102, Nr. 15, 15. April 1985, Seite 471, Nr. 130150j, Columbus, Ohio, US; M.L. STPHENSON et al.: "Immune interferon inhibits collagen synthesis by rheumatoid synovial cells associated with decreased levels of the procollagen mRNAs", & FEBS LETT. 1985, 180(1), 43-50 <br> * Insgesamt * <br><br> ---     -/- | 1-3,12 -17 | RECHERCHIERTE SACHGEBIETE (Int. Cl.4) <br><br> A 61 K |

Der vorliegende Recherchenbericht wurde für alle Patentansprüche erstellt.

| Recherchenort <br> DEN HAAG | Abschlußdatum der Recherche <br> 14-03-1986 | Prüfer <br> RYCKEBOSCH A.O.A. |
|---|---|---|

# 0205629

**Europäisches Patentamt**

**EUROPÄISCHER RECHERCHENBERICHT**

Nummer der Anmeldung

EP 85 10 7490

## EINSCHLÄGIGE DOKUMENTE

Seite 2

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | CHEMICAL ABSTRACTS, Band 100, Nr. 21, 21. Mai 1984, Seite 483, Nr. 172931d, Columbus, Ohio, US; I.F. BARINSKII et al.: "Successful therapy of autoimmune disease in NZB/W mice with interferon", & VOPR. VIRUSOL. 1984, 29(1), 38-42 * Insgesamt * | 1,9 | |

-----

RECHERCHIERTE SACHGEBIETE (Int. Cl.4)

Der vorliegende Recherchenbericht wurde für alle Patentansprüche erstellt.

| Recherchenort | Abschlußdatum der Recherche | Prüfer |
|---|---|---|
| DEN HAAG | 14-03-1986 | RYCKEBOSCH A.O.A. |